# FASCICULE DE BREVET EUROPEEN

(11) **EP 2 488 121 B1**
(45) Date de publication et mention de la délivrance du brevet: **11.03.2015**
(21) Numéro de dépôt: 10781934.4
(22) Date de dépôt: 11.10.2010
(51) Int. Cl.: A61B 17/80

(54) **Implant de stabilisation scapho-lunaire**
Implantat zur Stabilisierung des Ligamentum scapho-lunatum
Implant for scapho-lunate stabilisation

(30) Priorité: 13.10.2009 FR 0904894
(43) Date de publication de la demande: 22.08.2012
(73) Titulaire: BIOTECH ORTHO, 13300 Salon-de-Provence (FR)
(72) Inventeur: IMPELLIZZERI, Frédéric, F-13300 Salon de Provence (FR); HOUVET, Patrick, F-92100 Boulogne (FR)
(74) Mandataire: Marek, Pierre
(86) Numéro de dépôt international: PCT/FR2010/000681
(87) Numéro de publication internationale: WO 2011/045485

(56) Documents cités:
- EP-A1- 1 728 480
- US-A- 5 690 631
- US-A1- 2005 070 904
- US-A1- 2006 241 592
- US-A1- 2009 036 930

## Description

La présente invention concerne un implant de stabilisation scapho-lunaire.

Le scaphoïde et le semi-lunaire sont deux des huit os constituant le carpe. Lors d'une chute importante impliquant une réception sur le poignet, le ligament reliant ces deux os, appelé ligament scapho-lunaire, peut subir des lésions telles que déchirure, arrachement, .... La défaillance de ce ligament crée une instabilité qui entraîne une arthrose précoce.

Si cette lésion est prise en charge suffisamment tôt par un chirurgien, ce dernier peut effectuer une suture, ce qui n'est envisageable que si le ligament est encore vascularisé.

En revanche, dans le cas où la lésion n'est pas prise en charge rapidement, les dommages causés par les frottements de ces deux os peuvent être irréversibles, et il faut alors envisager l'arthrodèse, ou encore l'ablation des deux os.

On connaît aussi des dispositifs prothétiques permettant de remplacer le ligament scapho-lunaire.

Le plus souvent, ces dispositifs sont constitués de deux plaques destinées à être ancrées dans chacun des deux os à relier, ou fixées sur ces derniers, et d'une tige ou d'un fil permettant de faire la liaison desdites deux plaques.

L'inconvénient de tels dispositifs est qu'ils sont généralement constitués, en partie, d'éléments métalliques. Il y a donc un risque d'adhérences osseuses. En effet, lorsqu'un implant est présent dans l'organisme pendant une longue période, l'os a tendance à « repousser » sur l'implant et à recouvrir partiellement ce dernier. Il peut être alors impossible de le retirer dans son intégralité, ce qui peut poser des problèmes, notamment lors d'interventions chirurgicales postérieures.

En outre, les dispositifs actuels nécessitent, dans la plupart des cas, une immobilisation du poignet avec une attelle ou un plâtre pour une durée de 6 à 8 semaines, ce qui empêche toute mobilisation précoce et conduit à un enraidissement du poignet.

Le document EP 1 728 480 A1 divulgue un implant de stabilisation scapho-lunaire selon le préambule de la revendication 1.

Un but de la présente invention est de fournir aux chirurgiens orthopédiques, un implant de stabilisation simple, flexible et résistant, permettant de réaliser une liaison mécanique entre le scaphoïde et le semi-lunaire reproduisant le rôle du ligament scapho-lunaire.

Selon l'invention, ce but est atteint grâce à un implant de stabilisation apte à remplacer le ligament scapho-lunaire rompu ou à renforcer le ligament scapho-lunaire lésé, cet implant étant constitué d'une plaque de forme oblongue exécutée dans un matériau doté d'une capacité de déformation élastique, dont les extrémités sont munies d'au moins un trou permettant le passage de vis de fixation, et dont la partie centrale présente une ouverture délimitée par deux cotés opposés, un ressort de raideur prédéterminée reliant deux points distants, de préférence deux points opposés desdits cotés, ce ressort étant disposé suivant la diagonale dans ladite ouverture.

Le dispositif selon l'invention procure plusieurs avantages intéressants. Notamment :
- de fournir un implant qui remplit efficacement les fonctions du ligament scapho-lunaire,
- de permettre une mise en place rapide et précise de cet implant,
- d'autoriser des mouvements relatifs du scaphoïde et du semi-lunaire, c'est-à-dire un fonctionnement physiologique du poignet.

Selon un mode de mise en oeuvre avantageux, l'implant de stabilisation scapho-lunaire est de forme trapézoïdale, ou approximativement trapézoïdale.

Suivant une autre disposition caractéristique, l'implant selon l'invention comporte, dans sa partie centrale, une zone de déformation en forme de parallélogramme, ou approximativement de parallélogramme.

Selon un autre exemple de réalisation, chaque extrémité de l'implant de stabilisation scapho-lunaire est munie de deux trous de passage de vis.

L'implant selon l'invention est remarquable en ce qu'il est muni de trous permettant le passage de broches temporaires de maintien, disposés à proximité des trous de passage des vis de fixation.

Suivant une disposition importante de l'invention, la partie de l'implant constituant le ressort a une forme ondulée.

Selon un mode de réalisation avantageux, l'implant de stabilisation scapho-lunaire est réalisé d'une seule pièce, ce qui permet une grande simplification, notamment lors de la fabrication.

Selon un mode d'exécution préféré, l'implant de stabilisation scapho-lunaire est exécuté en tout matériau biocompatible présentant la robustesse et l'élasticité nécessaires.

Suivant un mode de réalisation avantageux, l'implant selon l'invention est exécuté en Polyétheréthercétone (PEEK).

Ce matériau possède des caractéristiques mécaniques très intéressantes car elles permettent une grande déformation tout en assurant une reprise de forme en position d'équilibre. Ce matériau est encore avantageux dans la mesure où il est radio transparent, et qu'il ne subit pas d'adhérences osseuses, il est donc possible de le retirer facilement, même après être resté en place dans l'organisme pendant plusieurs années.

Les buts, caractéristiques et avantages ci-dessus, et d'autres encore, ressortiront mieux de la description détaillée qui suit et des dessins annexés dans lesquels :

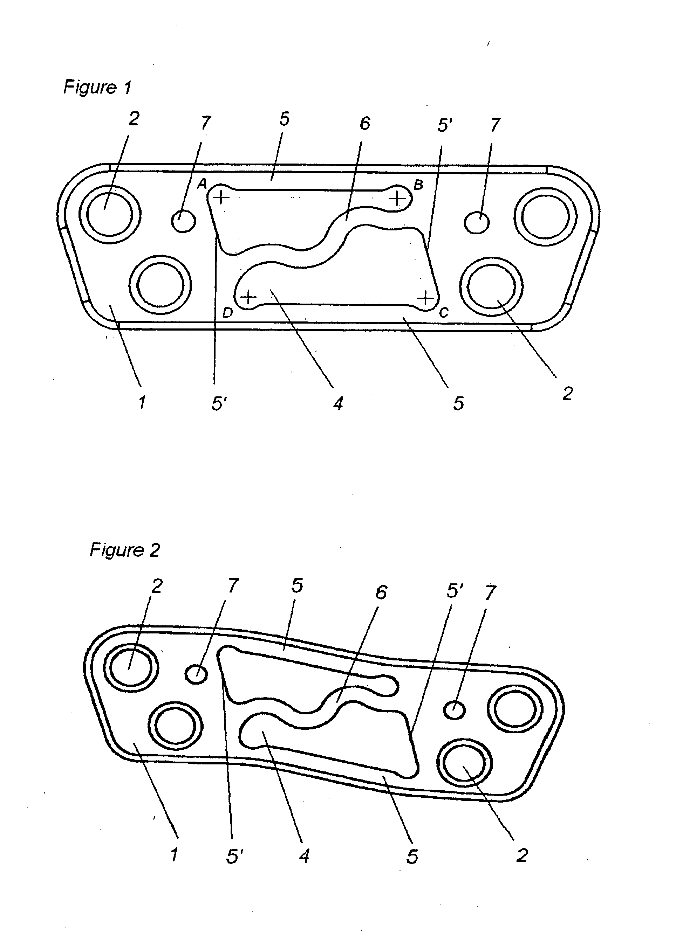

La figure 1 est une vue de dessus d'un premier exemple de réalisation de l'implant de stabilisation scapho-lunaire, illustré dans une position au repos.
La figure 2 est une vue analogue à la figure 1, montrant l'implant selon l'invention soumis à une charge.

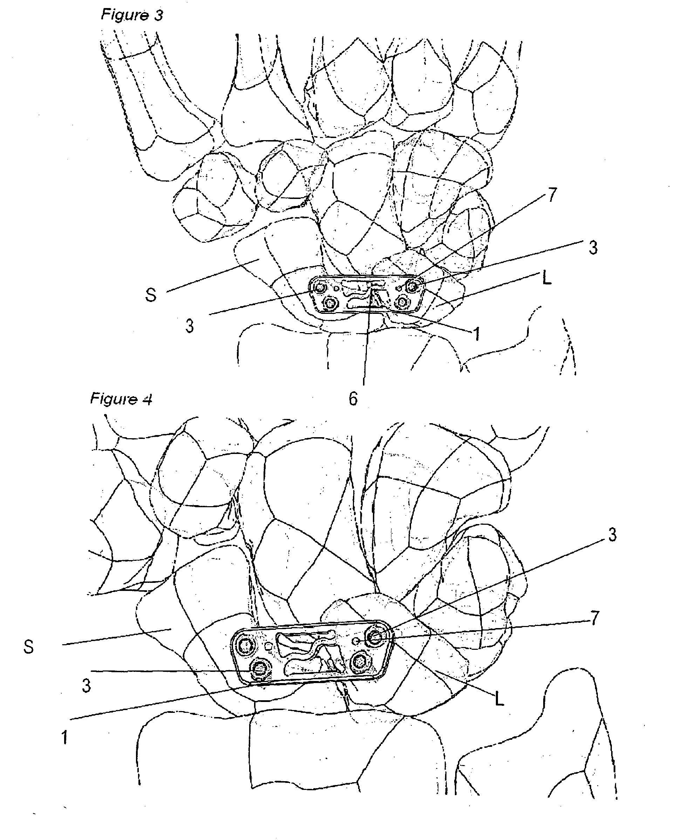

La figure 3 est vue en perspective illustrant le carpe de la main droite en face dorsale, muni d'un implant de stabilisation selon l'invention, en place sur le scaphoïde et le semi-lunaire.
La figure 4 est une vue analogue à la figure 3, et représentant un autre exemple de réalisation de l'implant selon l'invention.

On se réfère auxdits dessins pour décrire un exemple intéressant quoique nullement limitatif, de réalisation de l'implant de stabilisation scapho-lunaire, selon l'invention.

Cet implant de stabilisation scapho-lunaire est constitué d'une plaque 1 de forme oblongue exécutée dans un matériau doté d'une capacité de déformation élastique, dont les extrémités sont munies d'au moins un trou 2 permettant le passage de vis 3 de fixation, et dont la partie centrale présente une ouverture 4 délimitée par deux cotés 5 ou 5' opposés, un ressort 6 reliant deux points distants, par exemple deux points opposés desdits cotés 5 ou 5', ce ressort étant disposé selon la diagonale, dans ladite ouverture 4.

Selon l'exemple illustré, chaque extrémité de l'implant de stabilisation scapho-lunaire 1 est munie de deux trous 2 espacés de passage de vis 3 de fixation. Ces vis de fixation 3 sont, de manière préférée, du type à double filetage auto taraudant pouvant ainsi sculpter, automatiquement, lors de leur vissage, le taraudage des trous 2 qui sont avantageusement de forme conique.

La longueur de l'implant doit être suffisante pour permettre la fixation d'une de ses extrémités, sur le scaphoïde S, et la fixation de son autre extrémité, sur le semi-lunaire L.

Il peut par exemple mesurer de l'ordre de 20 mm de longueur, et 10 mm de largeur.

Destiné à être disposé sous la peau, l'implant de stabilisation selon l'invention a une faible épaisseur, de l'ordre de 1 à 1,5 mm, permettant ainsi à ce dernier d'être pratiquement indécelable sous la surface supérieure de la peau.

La forme générale de l'implant est dictée par l'anatomie des os.

Suivant un premier mode d'exécution représenté, l'implant de stabilisation scapho-lunaire est de forme trapézoïdale, ou approximativement trapézoïdale.

Selon un autre mode d'exécution illustré, l'implant selon l'invention a une forme de parallélogramme, ou approximativement de parallélogramme.

L'ouverture 4 présente dans la partie centrale de l'implant a, quant à elle, et de préférence, une forme de parallélogramme, ou sensiblement de parallélogramme. Cette partie centrale constitue une zone de déformation.

Selon la forme de l'ouverture 4, la longueur du ressort 6 varie. Ceci permet d'influer sur la raideur de ce ressort, et donc sur les déformations autorisées par l'implant 1.

D'autres formes peuvent néanmoins être envisagées, notamment pour permettre une utilisation main gauche, et une utilisation main droite.

De manière avantageuse, l'implant selon l'invention est remarquable en ce qu'il est muni d'au moins un trou 7 permettant le passage d'une broche temporaire de stabilisation, disposé à proximité des trous 2 de passage des vis de fixation 3.

En effet, avant de fixer l'implant scapho-lunaire 1 à l'aide des vis 3 dans chacun des deux os concernés S et L, le chirurgien doit d'abord stabiliser l'implant par rapport à ces os, puis effectuer des forages dans lesdits os pour la mise en place desdites vis 3.

De préférence, pour une meilleure stabilisation de l'implant, chacune des extrémités de l'implant est munie de deux trous 7.

Suivant l'exemple illustré, la partie de l'implant constituant le ressort 6 a une forme ondulée.

L'implant va donc se comporter comme un parallélogramme déformable dont la raideur est notamment conditionnée par la géométrie du ressort disposé sur une des diagonales.

Le mode de déformation de l'implant scapho-lunaire autorise un déplacement relatif de l'os S par rapport à l'os L tout en les maintenant en contact ou à faible distance.

De manière avantageuse, l'implant de stabilisation scapho-lunaire 1 est réalisé d'une seule pièce.

Ainsi, il est peut-être avantageusement réalisé selon des techniques d'injection plastique.

Selon un mode d'exécution préféré, l'implant de stabilisation scapho-lunaire 1 est exécuté en tout matériau biocompatible présentant la robustesse et l'élasticité nécessaires pour remplir sa fonction.

De préférence et avantageusement, l'implant 1 selon l'invention est exécuté en Polyétheréthercétone (PEEK).

## Revendications

1. Implant de stabilisation scapho-lunaire, qui est constitué d'une plaque (1) de forme oblongue exécutée dans un matériau doté d'une capacité de déformation élastique, dont les extrémités sont munies d'au moins un trou (2) permettant le passage de vis (3) de fixation, **caractérisé en ce que** la partie centrale de la plaque présente une ouverture (4) délimitée par deux cotés (5 ou 5') opposés, un ressort (6) reliant deux points distants desdits cotés (5 ou 5'), ce ressort étant disposé suivant la diagonale dans ladite ouverture.

2. Implant de stabilisation scapho-lunaire selon la revendication 1, **caractérisé en ce que** chacune de ses extrémités est munie de deux trous (2) de passage de vis (3).

3. Implant de stabilisation scapho-lunaire suivant l'une des revendications 1 ou 2, **caractérisé en ce qu'**il est de forme trapézoïdale, ou en forme de parallélogramme.

4. Implant de stabilisation scapho-lunaire selon l'une quelconque des revendications 1 à 3, **caractérisé en ce qu'**il comporte, dans sa partie centrale, une zone de déformation en forme de parallélogramme, ou approximativement de parallélogramme.

5. Implant de stabilisation scapho-lunaire suivant l'une quelconque des revendications 1 à 4, **caractérisé en ce qu'**il est muni d'au moins un trou (7) permettant le passage d'une broche temporaire de maintien, disposé à proximité des trous (2) de passage des vis de fixation (3).

6. Dispositif selon l'une quelconque des revendications 1 à 5, **caractérisé en ce que** la partie de l'implant constituant le ressort (6) a une forme ondulée.

7. Implant de stabilisation scapho-lunaire suivant l'une quelconque des revendications 1 à 6, **caractérisé en ce qu'**il est réalisé d'une seule pièce.

8. Implant de stabilisation scapho-lunaire selon l'une quelconque des revendications 1 à 7, **caractérisé en ce qu'**il est exécuté en tout matériau biocompatible présentant la robustesse et l'élasticité nécessaires.

9. Implant de stabilisation scapho-lunaire suivant l'une quelconque des revendications 1 à 8, **caractérisé en ce qu'**il est exécuté en Polyétheréthercétone (PEEK).

## Patentansprüche

1. Implantat zur Stabilisierung des Ligamentum scapholunatum bestehend aus einer Platte (1) mit einer längliche Form in einem Material mit einer Fähigkeit zur elastischen Verformung, deren Enden für den Durchgang von Schrauben (3) zur Befestigung mit zumindest einem Loch versehen ist (2) und wobei der Mittelabschnitt der Platte eine Öffnung (4) begrenzt von zwei gegenüberliegenden Seiten (5 oder 5 '), eine Feder (6), die zwei beabstandete Punkte der besagten Seiten (5 oder 5 ') verbindet, wobei die Feder entlang der Diagonalen in der besagten Öffnung angeordnet ist.

2. Implantat zur Stabilisierung des Ligamentum scapholunatum nach Anspruch 1,
**dadurch gekennzeichnet, dass** jedes ihrer Enden mit zwei Löchern (2) zum Durchgang von Schrauben (3) versehen ist.

3. Implantat zur Stabilisierung des Ligamentum scapholunatum nach einem der Ansprüche 1 oder 2, **dadurch gekennzeichnet, dass** es trapezförmig oder parallelogrammförmig geformt ist.

4. Implantat zur Stabilisierung des Ligamentum scapholunatum nach einem der Ansprüche 1 bis 3, **dadurch gekennzeichnet, dass** es eine Verformungszone, in seinem zentralen Abschnitt besitzt, in Form eines Parallelogramms oder annähernd eines Parallelogramms hat.

5. Implantat zur Stabilisierung des Ligamentum scapholunatum nach einem der Ansprüche 1 bis 4, **dadurch gekennzeichnet, dass** es mit zumindest einem Loch (7) für den Durchtritt eines temporären Haltestift in der Nähe der Löcher (2) für den Durchgang der Befestigungsschraube (3) ausgebildet ist.

6. Vorrichtung nach einem der Ansprüche 1 bis 5, **dadurch gekennzeichnet, dass** der Teil des Implantats, der die Feder (6) ausbildet, eine gewellte Form aufweist.

7. Implantat zur Stabilisierung des Ligamentum scapholunatum nach einem der Ansprüche 1 bis 6, **dadurch gekennzeichnet, dass** es einstückig ausgebildet ist.

8. Implantat zur Stabilisierung des Ligamentum scapholunatum nach einem der Ansprüche 1 bis 7, **dadurch gekennzeichnet, dass** es aus jedem biokompatiblen Material hergestellt ist, das die notwendige Festigkeit und Elastizität aufweist.

9. Implantat zur Stabilisierung des Ligamentum scapholunatum nach einem der Ansprüche 1 bis 8, **dadurch gekennzeichnet, dass** es aus Polyetheretherketon (PEEK) ausgebildet ist.

## Claims

1. A scapholunate stabilisation implant which is formed by a plate (1) of oblong shape and made of a material having a capacity for elastic deformation, the ends of which are provided with at least one hole (2) permitting the passage of fixing screws (3), **characterised in that** the central part of the plate has an opening (4) delimited by two opposite sides (5 or 5'), a spring (6) connecting two distant points of said sides (5 or 5'), said spring being disposed along the diagonal of said opening.

2. A scapholunate stabilisation implant according to claim 1 **characterised in that** each of its ends is provided with two holes (2) for the passage of screws (3).

3. A scapholunate stabilisation implant according to one of claims 1 and 2 **characterised in that** it is of trapezoidal shape or in the form of a parallelogram.

4. A scapholunate stabilisation implant according to any one of claims 1 to 3 **characterised in that** in its central part it has a deformation area in the form of a parallelogram or approximately a parallelogram.

5. A scapholunate stabilisation implant according to any one of claims 1 to 4 **characterised in that** it is provided with at least one hole (7) permitting the passage of a temporary holding pin, the hole (7) being disposed in the proximity of the holes (2) for the passage of the fixing screws (3).

6. A device according to any one of claims 1 to 5 **characterised in that** the part of the implant constituting the spring (6) is of an undulating shape.

7. A scapholunate stabilisation implant according to any one of claims 1 to 6 **characterised in that** it is made in one piece.

8. A scapholunate stabilisation implant according to any one of claims 1 to 7 **characterised in that** it is made of any biocompatible material having the necessary robustness and elasticity.

9. A scapholunate stabilisation implant according to any one of claims 1 to 8 **characterised in that** it is made of polyetheretherketone (PEEK).
